# EUROPEAN PATENT SPECIFICATION

(11) **EP 2 967 613 B1**
(45) Date of publication and mention of the grant of the patent: **22.04.2020**
(21) Application number: 14715762.2
(22) Date of filing: 10.03.2014
(51) Int. Cl.: A61B 17/221, A61B 17/3205, A61B 17/29, A61B 1/00

(54) **MEDICAL DEVICE HANDLES**
GRIFFE FÜR EINE MEDIZINISCHE VORRICHTUNG
POIGNÉES DE DISPOSITIF MÉDICAL

(30) Priority: 11.03.2013 US 201361776446 P
(43) Date of publication of application: 20.01.2016
(73) Proprietor: Boston Scientific Scimed, Inc., Maple Grove, MN 55311 (US)
(72) Inventor: JEMISON, Robert, Gosport, IN 47433 (US)
(74) Representative: Pfenning, Meinig & Partner mbB
(86) International application number: PCT/US2014/022746
(87) International publication number: WO 2014/164541

(56) References cited:
- EP-A1- 1 561 413
- EP-A1- 2 014 238
- US-A- 5 496 347
- US-A- 5 683 413
- US-B1- 6 210 398
- US-B1- 6 280 458

## Description

### Field of the Invention

Embodiments of the present disclosure relate generally to endoscopic medical devices suitable for use in medical procedures, and more particularly to improved actuation systems for such endoscopic medical devices.

### Background of the Invention

Minimally invasive medical procedures utilize instruments such as, e.g., endoscopes or other suitable introduction/access sheaths to, among other things, visualize a patient's internal cavities and organs and/or perform procedures within the patient. Such devices may be inserted into a patient's body through a natural opening or through a percutaneous incision, and such devices typically include a hollow steerable flexible tube with one or more channels therein to deliver therapy via a medical instrument or the like disposed within the one or more channels. The channels may also be used to provide irrigation, illumination, and/or suction.

EP 1 561 413 A1 describes an endoscopic therapeutic instrument which has a therapeutic section, a flexible transmission member which transmits operating driving forces to the therapeutic section by moving forwardly and reversely, and a sheath section in which the transmission member can move forwardly and reversely. A proximal side of this transmission member is connected to an operating tube section, and the therapeutic section is driven by forward and backward movements of the operating tube section. An endoscope having a channel into which the endoscopic therapeutic instrument is insertable, contains a mechanism for driving the sheath section and a mechanism for driving the operating tube section. The endoscope controls these mechanisms to cooperate with each other in controlling the endoscopic therapeutic instrument operations.

US 6 210 398 B1 describes a manipulating part of an endoscopic treatment tool, in which, to a cylindrical member having a first finger retaining member, there is slidably mounted a second finger retaining member slidably for remotely driving a distal end operating part using a sliding movement of the second finger retaining member along the cylindrical member. For the manipulating part, a stopper is added, which is movably provided on the cylindrical member, and which is fixed at an arbitrary position on the cylindrical member. The stopper is designed to restrict a length of the sliding movement of the second finger retaining member.

US 5 683 413 A describes a pair of forceps cups disposed on a distal end of an elongated guide tube which is opened and closed by remote control of a control mechanism disposed on a rear end of the guide tube through a wire within the guide tube. The control mechanism includes a holder having a rod portion, and a slider slidably disposed on the rod portion. A receiving member is mounted on a rear end portion of the wire at a forward position of the slider. This receiving member is biased backwardly by a compressed coil spring. The slider is not connected to the rear end portion of the wire. During the course of a forward movement of the slider, the slider contacts the receiving member and causes the wire to move forwardly through this receiving member. As a consequence, the pair of forceps cups is opened. When the slider is moved backwardly, the wire is pulled under the effect of the coil spring. Consequently, the pair of forceps cups is closed.

US 5 496 347 A describes a surgical instrument for treatment in the body cavity, in which an open-close member for treatment is rockably mounted by of a pivot pin on the distal end portion of a sheath to be inserted into the body cavity, and is connected to the distal end of a rod passed through the sheath, by a cam mechanism including a cam groove and a cam pin in engagement therewith. As the rod is moved by an operating handle, the cam mechanism is activated to open and close the open-close member for treatment. The component members of the cam mechanism have external shapes such that those portions on the proximal end side of the position near the pivot pin do not project from the sheath in every operating state.

EP 2 014 238 A1 describes an endoscopic treatment tool which is inserted to a body cavity endoscopically including a wire, a surgical section which is disposed on a first end of the wire and used for treatment inside the body cavity, and an operator section which is disposed on the second end of the wire and moves in conjunction with the wire so as to rotate by a rotary operation; and the movement of the operator section in conjunction with the wire is released upon exerting more than a predetermined torsional load on the wire.

US 6 280 458 B1 discloses a medical device, comprising an elongate shaft with an actuating member slidably disposed within the shaft, wherein a distal portion of the actuating member is operably coupled to an end-effector and wherein a handle is disposed at the proximal end of the elongate shaft, and wherein the handle comprises an elongate body portion defining a central bore therethrough, wherein the central bore includes a proximal opening, wherein a distal portion of the elongate body portion is operably coupled to the elongate shaft and an actuator operably coupled to the actuating member and slidably disposed within the central bore of the elongate body portion.

One exemplary environment where endoscopic devices are used is the urinary tract of a patient. Endoscopic devices are introduced into the urinary tract to perform, e.g., ureteroscopy, including stone extraction, stricture treatment, or stent placement. To remove bladder stones, e.g., a cystoscope or another suitable introduction sheath may be placed into a patient's bladder through the urethra, and subsequently, one or more medical instruments may be inserted through the cystoscope. These medical devices, e.g., a lithotripter and a retrieval device (e.g., a retrieval basket) may be inserted through the cystoscope to first break-up a urethral stone into smaller pieces and then extract the smaller pieces from the body, for example.

These various devices generally require at least one hand to manipulate a control element or to actuate an end-effector. Thus, operating multiple devices can pose difficulty if the operator or physician is required to simultaneously operate several devices. In other situations, some devices may need to be stabilized or held stationary while other devices are manipulated. Where one device cannot be left unattended during the operation of others, the most common solution is for an assistant to hold that device while another device may is operated. That solution is less than ideal.

In addition, the medical devices used to extract, e.g., urinary stones, typically include a sheath within which, e.g., a retrieval basket may be disposed. As is well known in the art, the retrieval may be expanded as it is deployed from the sheath. Subsequently, the basket may be manipulated or maneuvered to capture a stone. The basket with the stone captured therein may be then retracted until the stone is secured between a distal edge of the sheath and the basket, if the stone is too large to enter the sheath. Repeated use of the retrieval basket to capture multiple stones may exert compressive forces on the distal end of the sheath, thereby compressing the distal end of the sheath and shortening its effective length. As a result, the sheath may not fully cover the retrieval basket when the basket is fully withdrawn into the sheath in the closed position, making it difficult to capture smaller stones. Typically, an operator would then need to use a new device to continue removal of smaller stones.

Therefore, there exists a need for improved mechanisms for maintaining endoscopic medical devices when those devices are not being used during a procedure. A need also exists for compensating for sheath shortening, as described above.

### SUMMARY OF THE DISCLOSURE

Embodiments of the present disclosure provide a device for removably securing a proximal portion of a medical device to a suitable introduction sheath such as, e.g., an endoscope.

The invention is defined by claim 1. Preferred embodiments of the invention are defined by the dependent claims.

In accordance with an aspect of the present disclosure, a medical device handle may include an elongate body portion configured to be disposed at a proximal end of an elongate tubular member, wherein the elongate body portion defines a lumen therethrough. The medical device handle may also include an actuator slidably received within the lumen of the elongate body portion, wherein the actuator is configured to transition between a first longitudinal position and a second longitudinal position different than the first longitudinal position for operating an end-effector disposed at a distal end of the elongate tubular member. The medical device handle may also include a securing mechanism for securing the medical device handle to another medical device.

Various embodiments of the medical device handle may include one or more of the following features: the actuator may be operably coupled to an actuating member disposed within the elongate tubular member; a distal end portion of the actuating member may be operably attached to the end-effector; a location of the first longitudinal position may be adjustable relative to the elongate body portion; a proximal end portion of the actuating member may be fixedly secured to a proximal end of the actuator; rotation of the actuator relative to the elongate body portion alters a longitudinal position of the actuator relative to the elongate body portion; the securing mechanism may include a clip; the clip may include two clip arms projecting outwards to securely attach the medical device handle to the another medical device; the actuator may be biased to the first longitudinal position by a spring; the actuator may be secured to the elongate body portion by a generally cylindrical adjustable element; and the adjustable element may be secured to a compensator configured to be rotated by a user.

In another embodiment, a medical device may include an elongate sheath having a proximal end, a distal end, and a lumen extending therebetween, wherein at least a portion of an actuating member may be slidably disposed within the lumen, wherein a distal portion of the actuating member may be operably coupled to an end-effector, and wherein a handle may be disposed at the proximal end of the elongate sheath. The handle may include an elongate body portion defining a central bore therethrough, wherein the central bore may include a proximal opening, wherein a distal portion of the elongate body portion may be operably coupled to the elongate sheath, and wherein the elongate body portion may include a securing mechanism for securing the handle to a portion of another medical device. The handle may also include an actuator slidably disposed within the central bore of the elongate body portion, wherein the actuator may include a generally planar surface and an elongate tubular structure depending therefrom, wherein the actuating member may be configured to be received within the elongate tubular structure, wherein the actuator may be configured to transition between a first longitudinal position relative to the elongate body portion and a second longitudinal position different from the first longitudinal position.

Various embodiments of the medical device may include one or more of the following features: the planar surface may include a diameter larger than the diameter of elongate tubular structure; the actuator may be configured to move longitudinally relative to the elongate body portion upon rotation of the actuator relative to the elongate body portion; the actuator may be fixedly coupled to an adjustable body including threads on an outer surface thereof; a surface of the central bore may include threads for mating with the threads on the adjustable body; the actuating member may secured to the generally planar surface; the securing mechanism may include a clip; and the another medical device may include an endoscope.

In another embodiment, a medical system may include an introduction sheath including a proximal end, a distal end, and a plurality of working channels extending therebetween, wherein the proximal end of the introduction sheath may include a hub having a port in communication with at least one of the plurality of working channels. The medical device may also include an elongate tubular member defining a proximal end, a distal end, and a lumen therebetween, wherein the proximal end of the elongate tubular member may be operably coupled to a handle, wherein a distal portion of the elongate tubular member may be configured to be disposed within the port. The handle may include an elongate body disposed at the proximal end of the elongate tubular member, wherein the elongate body portion may define a lumen therethrough. The handle may also include an actuator slidably received within the lumen of the elongate body, wherein the actuator may be configured to travel between a first proximalmost position relative to the elongate body and a second distalmost position relative to the elongate body, wherein rotation of the actuator may move one of the first proximalmost and second distalmost positions. The handle may further include a securing mechanism for securing the medical device handle to the hub.

Various embodiments of the medical system may include one or more of the following features: the securing mechanism may include a clip; and an actuating element having a proximal end and a distal end, wherein the proximal end may be secured to the actuator and the distal end may be secured to an expandable basket.

Additional objects and advantages of the disclosure will be set forth in part in the description which follows, and in part will be obvious from the description, or may be learned by practice of the disclosure. The objects and advantages of the disclosure will be realized and attained by means of the elements and combinations particularly pointed out in the appended claims which define the present invention.

It is to be understood that both the foregoing general description and the following detailed description are exemplary and explanatory only and are not restrictive of the disclosure, as claimed.

### BRIEF DESCRIPTION OF THE DRAWINGS

The accompanying drawings, which are incorporated in and constitute a part of this specification, illustrate exemplary embodiments of the present disclosure and together with the description, serve to explain the principles of the disclosure.
**FIG. 1** is a schematic view of an exemplary device.
**FIG. 2A** is a schematic view of an exemplary device, according to an embodiment of the present disclosure.
**FIG. 2B** is an exploded view of the exemplary device of FIG. 2A.
**FIG. 3** is a perspective view of the exemplary device of FIG. 2A removably secured to an introduction sheath.
**FIG. 4** is a perspective view of an alternative device, according to another embodiment of the present disclosure.
**FIG. 5** is a side view of a further alternative device, according to another embodiment of the present disclosure.
**FIG. 6** is a detail schematic view of the exemplary device of FIG. 2A, illustrating operation of a portion of that embodiment, according to the present disclosure.

### DESCRIPTION OF THE EMBODIMENTS

Reference will now be made in detail to embodiments of the present disclosure, an example of which is illustrated in the accompanying drawings. Wherever possible, the same reference numbers will be used throughout the drawings to refer to the same or like parts.

### Overview

Embodiments of the present disclosure relate to systems used to secure one or more actuation handles during the course of a minimally invasive surgical or diagnostic procedure. In one embodiment, an actuation handle of a medical device may be provided with a securing mechanism (e.g., a clip), allowing an operator to removably attach the actuation handle to a convenient, stable location, such as, e.g., a port body located on an endoscope or other suitable introduction sheath. Various configurations of the medical device articulation, structure, and function are described in the embodiments of the disclosure. Further, as used in this disclosure, "distal" refers to a position or direction further from a user, and "proximal" refers to a position or direction opposite "distal" and closer to the user.

### Exemplary Embodiments

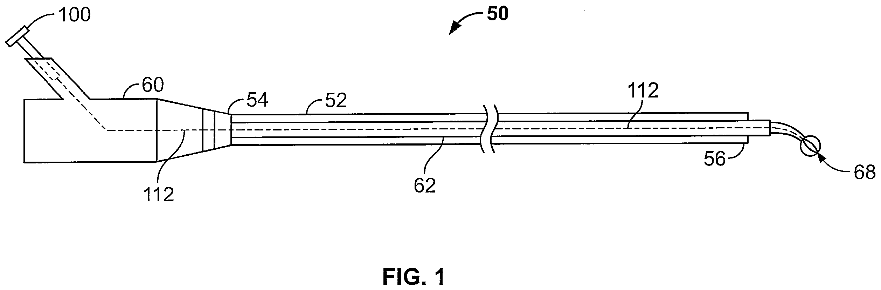

For purposes of illustration only, an exemplary medical instrument capable of being used with the actuation handle of the present disclosure will be described. This exemplary description should not be limiting, and those of ordinary skill in the art will understand that the principles disclosed herein may be used with any suitable medical instrument. As shown in Fig. 1, the exemplary medical device 50 may include an elongate sheath 52 having a proximal end 54, a distal end 56 and a lumen 62 extending therebetween. At its distal end 56 the sheath 52 may include an opening (not shown) in communication with the lumen 62. In addition, a handle body 60 may carry an actuator, such as, e.g. actuator 100, disposed at the proximal end 54 of the elongate sheath 52. The actuator 100 may be operably coupled to an actuating member 112. The actuating member 112 may extend distally from the actuator through the lumen 62 of the elongate sheath 52. An end-effector 68 such as, e.g., a snare, scissors, forceps, retrieval basket, needle, etc., may be operably coupled to a distal end of the actuating member 112. The end-effector 68 may be configured to transition between a first configuration and a second configuration. In the first configuration, the end-effector 68 may be disposed within a distal portion of the elongate sheath 52 in a collapsed configuration. In the second configuration, the end-effector 68 may be extended out of the elongate sheath 52 and may be expanded for use. The end-effector 68 may be self-expanding or may expanded by another device.

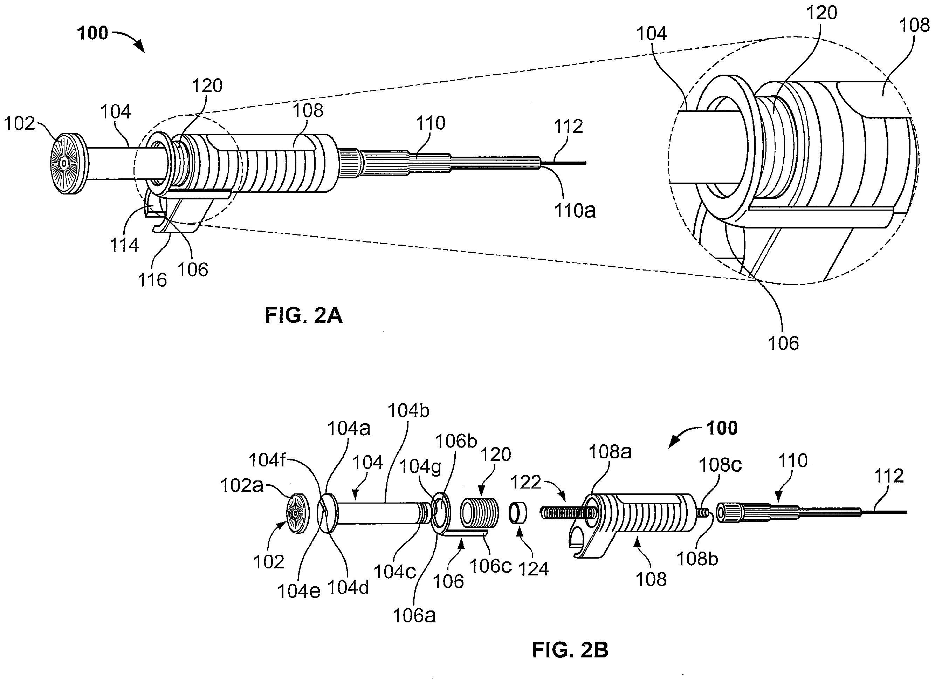

FIGS. 2A and 2B are schematic and exploded views, respectively, of an actuation handle 100, according to an embodiment of the present disclosure. Actuation handle 100 may include a handle body 108, which may be generally cylindrical, trapezoidal, rectangular, or in an appropriate form, and which may be configured to slidably receive an actuator 104 into a central bore 108a.

For purposes of illustration only, further discussion of actuation handle 100 and associated components will presume a system aimed at retrieving stones or other unwanted material from a patient's urinary system. In such a system, actuation handle 100 could be employed, for example, to open and close a snare or basket retrieval device. It will be understood that stone retrieval is only one of a great many possible applications for actuation handle 100, and none of those other applications is foreclosed by the chosen example. That is, actuation handle 100 may be used in conjunction with any suitable endoscopic or laparoscopic medical instrument.

Although the depicted embodiments indicate that handle body 108 includes a substantially cylindrical form, handle body 108 may include any suitable configuration. For example, handle body 108 may include a square, rectangular, or triangular cross-sectional configuration. Still further configurations could include an ellipsoidal cross-section in a portion of handle body 60 adapted for gripping by a human hand, combined with a circular cross-section in other portions of the device. Those of skill in the art will be able to determine the operational needs for a given handle body and the design needed to accomplish those goals. Further, although the depicted embodiments describe central bore 108a as having a substantially circular cross-sectional configuration, central bore 108a may include any suitable configuration. For example, central bore 108a could have an ovoid shape if it were desirable to minimize one dimension, such as thickness. If it were desired to prevent rotation, a cross-section having edges, such as a rectangular, square, or octagonal cross-section could be employed for central bore 108a. In some embodiments, a proximal portion of central bore 108a may include threads to correspond with threads on an outer surface of adjustable body 120, as discussed in greater detail below.

Actuator 104 may be generally sized to be slidingly received within handle body 108, as discussed below in greater detail. If desired, a proximal portion, such as, e.g., a proximal end, of actuator 104 may be widened into a flange 104a, adapted for comfortable fit to an operator's thumb. More particularly, actuator 104 may include a generally elongate structure 104b configured to be received within central bore 108a. Although the depicted embodiments show elongate structure 104b as having a generally cylindrical shape, elongate structure 104b may have any suitable shape, form, and/or configuration corresponding to central bore 108a. In addition, elongate structure 104b may include a lumen (not shown) extending therein. The walls of the lumen may be provided with any suitable coating to, e.g., facilitate movement of the actuating member 112 therein. In addition, the outer walls of elongate structure 104b may also include a suitable coating for improving movement within central bore 108a. The lumen may be in communication with an opening (not shown) at the distal end of elongate structure 104b. The edges of the opening may be chamfered and or rounded to avoid undue wear and tear on actuating member 112. At its proximal end, the lumen may be in communication with an opening 104d disposed on flange 104a. Opening 104d may include any suitable configuration. For example, opening 104d may be substantially circular. In some embodiments, opening 104d may include a dimension that is different than a dimension of the opening in the distal end of elongate structure 104b. For example, opening 104d may include a diameter that is smaller than the diameter of the opening in the distal end of elongate structure 104b.

Opening 104d may be in communication with a slot 104e. Slot 104e may include any suitable configuration. For example, slot 104e may extend radially away from opening 104d. In addition, slot 104e may extend through the entire thickness of flange 104a. In some embodiments, however, slot 104e may only extend partly through the thickness of flange 104a. Further, slot 104e may extend from opening 104d all the way to the end of flange 104a. Alternatively, slot 104e may only extend a desired distance away from opening 104d. Although the depicted embodiment illustrates slot 104e as having a substantially linear configuration, slot 104e may include any suitable curves and/or turns. In some embodiments, slot 104e may terminate in a recess 104f disposed in a radial edge of flange 104a. The recess 104f may include any suitable configuration. For example, in an embodiment, recess 104f may include a semi-circular configuration. In another embodiment, for example, recess 104f may include a substantially triangular configuration. Like slot 104e, recess 104f may either extend all the way through flange 104a or partly through the thickness of flange 104a. The functionality and purpose of opening 104d, slot 104e, and recess 104f will be discussed in greater detail below.

Moreover, a cap 102 may be secured to flange 104a. Cap 102 may be configured to correspond to flange 104a in any of a number of ways. For example, cap 102 may have a diameter that is substantially similar to the diameter of flange 104a. In addition, a distal face of cap 102 may have one or more geometric features that complement one or more of opening 104d, slot 104e, and/or recess 104f. For example, cap 102 may include a slot (not shown) that, when cap 102 is mated to flange 104a, cooperates with slot 104e to define a lumen or channel. Cap 102 may be secured to flange 104a by any suitable means. For example, cap 102 may be secured to flange 104a via an adhesive. Further, a proximal face 102a of cap 102 may include any suitable marking. For example, face 102a may include a logo of a manufacturer. Cap 102 and flange 104a may be also made of one-piece construction.

Further, a distal portion of elongate structure 104b may include a plurality of circumferential ribs 104c. Ribs 104c may include screw threads for cooperating with threads disposed on an inner surface of keeper 124, as discussed in greater detail below. In some embodiments, ribs 104c may be configured as circumferential indentations. As will be discussed in greater detail below, ribs 104c may be configured to secure keeper 124 to elongate structure 104b.

The construction, fabrication, and materials of actuation handle 100 are generally conventional. Typical materials include polymers, such as ABS (acrylonitrile butadiene styrene), polycarbonate, or high-density polyethylene. The materials and constructional details are sufficiently well-known to those of ordinary skill in the art and no further explanation is required here.

In operation, a user may actuate actuator 104 to effect operation of end-effector 68. For example, pushing actuator 104 towards to handle body 108 such that elongate structure 104b is received into central bore 108 may cause end-effector 68 to be advanced distally out of sheath 56. In embodiments where end-effector 68 is configured to self-expand, for example, end-effector 68 may begin to expand as soon as some or all of it begins to leave the confines of sheath. In addition, pulling actuator 104 proximally away from handle body 108 may cause end-effector 68 to be withdrawn into a distal end of sheath 56. In some embodiments, one or more of the actuator 104 or end-effector 68 may be resiliently biased by, e.g., a suitable spring or spring-like mechanism, as desired. For example, in one embodiment, actuator 104 may be biased to a proximalmost position so that end-effector 68 is biased into sheath 56. In another embodiment, actuator 104 may be biased to a distalmost position so that end-effector 68 is biased out of sheath 56. Such biasing of either actuator 104 or end-effector 68 may facilitate one-handed operation by an actuator as described in greater detail below. In addition, or alternatively, to moving end-effector 68 proximally and/or distally relative to sheath 56, actuator 104 may be configured to rotate end-effector 68 about a longitudinal axis of sheath 56. For example, rotating actuator 104 clockwise or counterclockwise may cause end-effector 68 to be correspondingly rotated.

Further, those of ordinary skill in the art will recognize that the principles described herein may be used to move sheath 25 proximally and distally relative to end-effector 68.

With renewed reference to Fig. 2A, for example, a proximal end portion of actuator 104, such as, e.g., cap 102, flange 104a, or elongate structure 104b, may be configured to facilitate one-handed operation by a user. More particularly, a proximal end portion of actuator 104 may include one or more geometric configurations to facilitate secure operation by, e.g., a user's thumb. Such geometric configurations may include but are not limited to a ring or ring-like structure to allow a user's thumb or other finger's to move the actuator 104 proximally or distally. Other geometric configurations may include a notch, cutout, or other indentation for engaging a portion of a user's thumb or finger. Further, a proximal end portion of actuator 104 may include a knob, lever, hook, bump, or other protrusion(s) configured to be engaged by a user to facilitate one-handed actuator, which may include advancing, withdrawing, or rotating actuator 104 to rotate end-effector 68.

Elongate structure 104b may include suitable markings or other indicia thereon for communicating to a user whether and to what degree end-effector 68 has been extended out of sheath 56. For example, an outer wall of elongate structure 104b may include a plurality of graduated markings that correspond to various distances that end-effector 68 may protrude from sheath 56.

As alluded to above, an actuation member 112 may extend distally from actuator 104. More particularly, a proximal portion of actuating member 112 may be received within the lumen of elongate structure 104b through the opening in the distal end thereof. The proximal portion of actuating member 112 may extend through elongate structure 104b and out of opening 104d. There, a proximal end of actuating member 112 may be bent a first time so that it may be at least partially received within slot 104e. The proximalmost end of actuating member 112 then may be bent a second time so that it is received within recess 104f. Subsequently, cap 102 may be secured to flange 104a, thereby fixedly securing the proximal portion of actuating member 112 to flange 104a.

The actuation member 112 may exit handle body 108 at its distal end through an opening 108b formed in the distal tip thereof. Actuation member 112 may extend onward through the elongate sheath 52 to or beyond the elongate sheath's distal tip. The actuation member 112 may move distally and proximally relative to handle body 108 (and sheath 52) under control of the actuator 104 to operate an end-effector at the distal end of the elongate sheath 52. Actuation member 112 may include a control rod or one or more wires, which may be braided together. Depending upon the specific application to which it is put, the actuation member 112 may be sufficiently stiff to apply forces to an end-effector, for example, without kinking. In other applications, the only significant force to be applied to actuation member 112 may be a pulling force, requiring tensile strength but not stiffness. Typical materials employed for fabricating actuation member 112 may include stainless steels, nitinol, or suitable polymers.

The distal end of actuation handle 100 may include a strain relief member 110, threaded for attachment to a male threaded projection 108c on the distal end of handle body 108. Strain relief member 110 may be disposed between handle body 108 and the elongate sheath 52 discussed above. In some embodiments, strain relief member 110 may be made integral with one or more of handle body 108 and the elongate sheath 52. Strain relief member 110 may have a central lumen 110a through which the actuation member 112 may extend. In addition, the strain relief member 110 may taper radially inward, from a relatively larger diameter at its proximal end to a relatively smaller diameter at its distal end. If desired, a ribbed profile may be adopted, with three or four ribs running lengthwise on the outer surface of the strain relief member 110. That structure would in part enhance bending stiffness. Any convenient attachment mechanism can be substituted for the male and female threads. For example, a Luer-lock, snap-fit, or similar mechanism will be suitable. The attachment must exhibit enough strength to allow a bending moment to be applied to the strain relief member 110, distal of the handle body 108, without causing the strain relief member 110 either to plastically deform or to separate at the attachment mechanism. That property allows the handle body to undergo a certain amount of strain without damaging the actuation wire 112 or kinking the elongate sheath 52 extending distally from the strain relief 110.

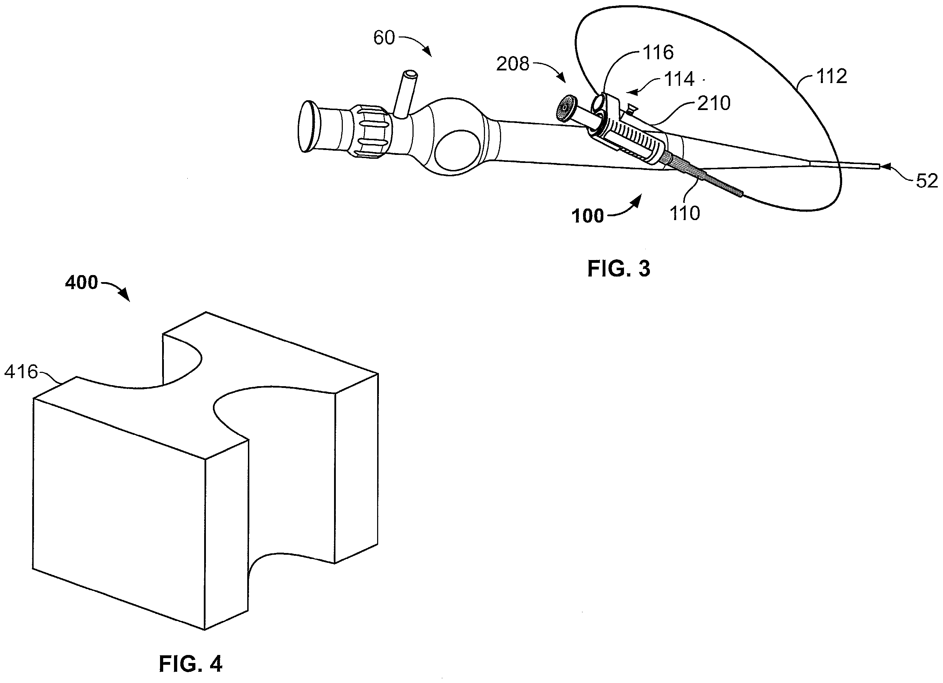

In an embodiment discussed in connection with FIG. 3, below, it will be observed that an actuation member 212 is carried in a loop before entering a port 210. This arrangement results in a moment being applied to the distal portion of actuator 100. As shown, that moment may be absorbed by strain relief 110, which may flex or bend under the applied forces. The strain applied to, e.g., actuation member 212 is thus spread over the length of strain relief 110 rather than being concentrated at the single point where actuation member 212 enters the handle body 108. In this manner, kinking may be avoided. In a similar fashion, kinking of sheath 52 also may be avoided.

Components of actuation handle 100 carried within handle body 108, best seen in FIG. 2B, enable a number of important functions. For example, a coil spring 122 may be carried within handle body 108. The coil spring 122 may be disposed about actuation member 112. By riding on actuation member 112, coil spring 122 may remain in position, where it biases actuator 104 to its fully proximal position. This fully proximal position may correspond to the closed or collapsed configuration of the end-effector. Thus, in operation, a user may depress actuator 104 distally relative to handle body 108 to deploy and/or expand an end-effector such as, e.g., a retrieval basket. Once the user removes the force applied to move actuator 104 distally relative to body 108, coil spring 122 may urge actuator 104 proximally, thereby causing actuating member 112 to pull the exemplary retrieval basket proximally into the elongate sheath 52, which may cause the basket to collapse about a captured stone.

Keeper 124 may be generally annular in form, with an outside diameter sized to fit into central bore 108a and an inner diameter sized to fit over elongate structure 104b. Keeper 124 may be configured to cooperate with ribs 104c to retain keeper 124 on a distal portion of elongate structure 104b. To that end, keeper 124 may be provided with attachment means, such as threads, a snap-fit, or a key mechanism, as known in the art. In the illustrated embodiment, keeper 124 is provided with a snap-fit mechanism, adapted to engage ribs 104c formed in the distal portion of actuator 104. In operation, once compensator 106 and adjustable body 120 are received onto elongate structure 104b, as discussed in greater detail below, keeper 124 may be secured to ribs 104c, thereby preventing elongate structure 104b from becoming disengaged from compensator 106 and adjustable body 120. In embodiments where keeper 124 includes threads, keeper 124 may be screwed onto the distal end 104g of elongate structure 104b. In other embodiments, keeper 124 may be secured to the distal end 104g of elongate structure 104b by, e.g., a press fit or friction fit. An adhesive may also be used to secure keeper 124 to distal end 104g. As depicted in the figures, the entire assembly may be then received within central bore 108a.

Adjustable body 120 may be sized to fit into the central bore 108a of handle body 108, and its outer surface may include a plurality of protrusions, such as, e.g., threads, in accordance with the present claimed invention. As alluded to above, adjustable body may be removably secured to body 108 by screw fit to central bore 108a. In some embodiments, adjustable body 120 may be formed of a resilient material, so that it may be securely disposed within the central bore 108a of handle body 108. Further, as shown in Fig. 1B, adjustable body 120 also has a central lumen, sized to receive a portion of actuator 104 in a smooth sliding fit, as discussed above.

With continuing reference to Fig. 2B, compensator 106 may include a washer-like portion 106a having a central opening 106b and an arm 106c extending distally therefrom. The central opening 106b may be configured to receive elongate structure 104b therethrough. Although the depicted embodiments indicate that central opening 106b includes a substantially circular configuration, central opening 106b may include any suitable configuration and/or shape. In addition, arm 106c may be shaped for convenient manipulation by an operator's thumb. Suitable shapes for that purpose might be triangular, with an apex of the triangle extending radially, or a rounded shape having a knurled surface for improved gripping. Once compensator 106 and adjustable body 120 are disposed about elongate structure 104b, keeper 124 may be secured to a distal end portion of elongate structure 104b to secure compensator 106 and adjustable body 120 on elongate structure 104b. Compensator 106 may be secured to adjustable body 120 by any suitable means. In one embodiment, e.g., compensator 106 may be fixedly secured to adjustable body 120 by an adhesive. In other embodiments, compensator 106 and adjustable body 120 may be formed from a one-piece configuration.

Compensator 106 allows an operator to adjust the position of actuator 104 relative to body 108. As noted above, repeatedly capturing stones in, e.g., a retrieval basket, may exert compressive forces on the distal end of the elongate sheath 52 of the medical device. These compressive forces, over time, may cause the elongate sheath 52 to shrink relative to the actuating member 112 disposed therein. As a result, the end-effector (e.g., a retrieval basket) of such a medical device may no longer be fully received within the sheath 52. If this happens, the retrieval basket may become ineffective at capturing relatively smaller stones. Consequently, the device may have to be discarded and a new device may need to be inserted into the patient. Compensator 106 attempts to address this issue by allowing an operator to adjust the relative position of the actuating member 112 within the elongate sheath 52.

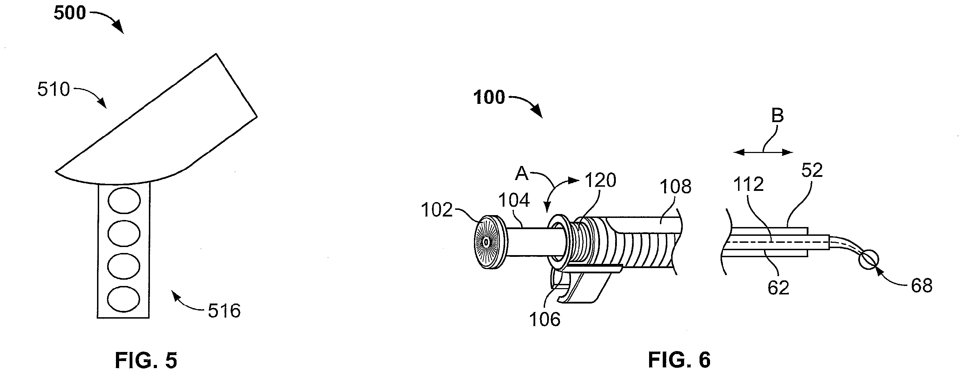

Turning now to Fig. 6, an operator may adjust the position of actuating member 112 relative to the elongate sheath 52 by rotating the compensator 106 in the directions of arrow A. Such rotation will cause adjustable body 120 to rotate relative to body 108. Since adjustable body 120 is threaded to body 108, rotation of adjustable body 120 will result in longitudinally advancing adjustable body 120 into or out of central bore 108a relative to body 108. This longitudinal movement, as a result of the connections between actuator 104 and actuating member 112, will move the distal end-effector 68 relative to the elongate sheath 52 in the directions shown by arrow B (shown in Fig. 6), thereby allowing an operator to adjust the end-effector position within the elongate sheath 52. That is, rotating adjustable body 120 in a first direction of arrow A may cause actuating member 112 to move end effector 60 proximally relative to elongate sheath 52. Alternatively, rotating adjustable body 120 in a second direction of arrow A may cause actuating member 112 to move end effector 60 distally relative to elongate sheath 52.

Clip 114 may extend laterally away from a proximal portion of handle body 108, with two clip arms 116 projecting outward, adapted to removably but securely attach actuation handle 100 to, e.g., an endoscope handle 60 (FIG. 3). In embodiments where a mounting device for the endoscope handle may be provided, positions on the handle may be a convenient choice as an object for attachment.

FIG. 3 depicts an actuation handle 100 mounted on a handle 60 of, e.g., an endoscope or other suitable introduction sheath. There, clip 114 is able to attach to a port body 210, which may be located in the distal portion of the handle 60. In the illustrated embodiment, clip 114 and clip arms 116 are particularly adapted to fit on port body 210. For example, clip arms 116 may be formed of a resilient material, allowing them to expand outward to slide over an object, e.g., port body 210, and then clamp onto that object with sufficient force to retain actuator handle him 100 stably in position. In other embodiments, clip 114 may be provided with adjustable adaptations of clip arms 116 which could accommodate a wide variety of clamping objects. Those of skill in the art are capable of adapting mechanisms from conventional graspers or clamps to serve in the described functions. In other embodiments, an attachable mounting object could be provided.

Actuation member 112 extends distally from the actuation handle 100. The geometry of the handle 60 and actuation handle 100 may require that actuation member 112 form a loop and then enter handle 60 through port 208. From there, the actuation member 112 enters introduction sheath 52, as described above.

Clip 114 can be fashioned in any size or shape that appears convenient for attaching actuation handle 100 to an object likely to be available to the physician during a procedure. Alternatively, an attachment mechanism roughly like clip 114 could be incorporated into the manufacture of devices similar to endoscopic device handle 60. The range of such mechanisms is limited only by the equipment likely to be in the vicinity during the conduct of a procedure employing minimally invasive equipment.

In yet further embodiments, illustrated in FIG. 4, e.g., a double-ended clip 400 may be employed. The double-ended clip 400 is generally cubical, having general dimensions similar to those of clip 114. Here, however, pairs of clip arms 416 extend from both sides of the structure. Thus, a conventional device, such as device 50 of FIG. 1, can be attached to a suitable structure, such as port body 210 of handle 60 shown in FIG. 3. This arrangement allows one to achieve the advantage of stabilizing an actuation handle without requiring a specially manufactured device already including attachment mechanisms. Of course, dimensions of both sets of clips must be adapted to particular target structures, such as standard actuator handles and port sizes or similar stabilizing structures to which an actuator handle can be attached. The clip arms 416 also may be suitably adjustable to accommodate variations in instrument sizes.

FIG. 5 is a side view of an attachable mounting point 500 , in accordance with yet another embodiment of the present disclosure. Situations may arise in which a convenient mounting point for an actuation handle 100 is not available in a particular situation. One solution would be to employ mounting point 500, which includes a body portion 510, shaped to emulate a device to which the particular actuating handle, such as actuating handle 100, is already adapted. One choice, for example, would be to emulate an endoscopic device port, to which actuation handle 100 may already be adapted for attachment. Attachment can be accomplished by using mounting strap 516 extending from the base portion of body portion 510. Mounting strap 516 can be a single strap that includes a fastening device, or multiple straps with provisions for fastening to one another. The fastening portion can be any of the many well-known devices available to the art. Once the mounting point 500 is attached to any of the many stabilizing points mentioned above, that point can be employed to carry an actuating handle 100.

The embodiments disclosed herein are configured to use with any suitable medical device configured for insertion into a patient's body. In one embodiment, for example, actuation handle 100 may be used with a device configured for retrieving unwanted material (e.g., tissue, debris, kidney stones, biliary stones, and/or the like) from within a patient. The device may include a retrieval device (e.g., an end-effector 68) configured as an expandable basket.

In an exemplary method of use, the retrieval device may be advanced to a desired location within a patient with the aid of an endoscope or a suitable introduction sheath. Once appropriately positioned, actuator 104 may be pushed distally relative to handle body 108 to advance the retrieval device out of sheath 56 so that it may be disposed adjacent material targeted for removal from within the patient. If necessary, actuator 104 may be rotated so that the retrieval device may be appropriately positioned for capturing or otherwise retaining the targeted material. Subsequently, actuator 104 may be withdrawn proximally to withdraw the retrieval device into sheath 56, thereby capturing the targeted material for removal. If the targeted material is small enough to fit inside of sheath 56, the entire retrieval device may be removed from within the patient. If, however, the targeted material is too large, the targeted material may be trapped between a distal endface of sheath 56 and the retrieval device. If the actuator 104 cannot be withdraw far enough in the proximal direction to ensure the targeted material engages the distal endface of sheath 56, the operator of the device may rotate compensator 106 to adjust a position of actuator 104 relative to handle body 108, as described above, so that actuation member 112 and the retrieval device may be moved relative to sheath 56.

The embodiments disclosed herein contemplate one-handed operation by a user. Accordingly, the actuator 104 may be moved distally, proximally, rotated, and otherwise manipulated with the aid of suitable geometric configurations disposed at a proximal end portion of actuator 104. In addition, should a user require both hands for another task, the handle body 108 includes a clip 114 for securing the actuation handle to a portion of a handle of the introduction sheath, as explained above.

Embodiments of the present disclosure may be used in any medical or non-medical procedure. In addition, at least certain aspects of the aforementioned embodiments may be combined with other aspects of the embodiments, or removed, without departing from the scope of the disclosure.

Other embodiments of the present disclosure will be apparent to those skilled in the art from consideration of the specification and practice of the embodiments disclosed herein. It is intended that the specification and examples be considered as exemplary only, with a true scope of the invention being indicated by the following claims.

## Claims

1. A medical device (50), comprising:
an elongate sheath (52) having a proximal end (54), a distal end (56), and a lumen (62) extending therebetween, wherein at least a portion of an actuating member (112) is slidably disposed within the lumen (62), wherein a distal portion of the actuating member (112) is operably coupled to an end-effector (68), and wherein a handle (100) is disposed at the proximal end (54) of the elongate sheath (52), and wherein the handle (100) comprises:
an elongate body portion defining a central bore (108a) therethrough, wherein the central bore (108a) includes a proximal opening, wherein a distal portion of the elongate body portion is operably coupled to the elongate sheath (52); and
an actuator (104) operably coupled to the actuating member (112) and slidably disposed within the central bore (108b) of the elongate body portion, wherein the actuator (104) includes a generally planar surface and an elongate tubular structure (104b) depending therefrom and configured to be received within the central bore (108a), wherein the actuating member (112) is configured to be received within the elongate tubular structure (104b), wherein the actuator (104) is configured to transition between a first longitudinal position relative to the elongate body portion and a second longitudinal position different from the first longitudinal position,
wherein the actuator (104) is fixedly coupled to an adjustable body (120) including threads on an outer surface thereof and wherein a surface of the central bore (108a) includes threads for mating with the threads on the adjustable body (120).

2. The medical device (50) of claim 1, wherein the planar surface includes a diameter larger than a diameter of elongate tubular structure (104b).

3. The medical device (50) of claim 1, wherein the actuator (104) is configured to move longitudinally relative to the elongate body portion upon rotation of the actuator (104) relative to the elongate body portion.

4. The medical device (50) of claim 1, wherein the actuating member (112) is secured to the generally planar surface.

5. The medical device (50) of claim 1, wherein the elongate body portion includes a securing mechanism for securing the handle (100) to a portion of another medical device, and wherein the securing mechanism includes a clip (114).

6. The medical device (50) of claim 1, wherein the another medical device includes an endoscope.

7. The medical device (50) of claim 1, wherein a proximal end portion of the actuating member (112) is fixedly secured to a proximal end of the actuator (104).

8. The medical device (50) of claim 5, wherein the clip (114) has two clip arms (116) projecting outwards to securely attach the medical device handle to the another medical device.

9. The medical device (50) of claim 1, wherein the actuator (104) is biased to the first longitudinal position by a spring (112).

10. The medical device (50) of claim 1, wherein the elongate structure (104b) of the adjustable body (120) has a configuration corresponding to the central bore (108a) and a lumen extending therein to receive the actuating member (112).

11. The medical device (50) of claim 1, wherein the adjustable body (120) is secured to a compensator (106) configured to be rotated by a user.

12. The medical device (50) of claim 11, wherein the compensator (106) and the adjustable body (120) are received onto the elongate structure (104b) of the adjustable body (120).

13. The medical device (50) of one of claims 11 or 12, wherein the compensator (106) includes a washer-like portion (106a) having a central opening (106b) and an arm (106c) extending distally therefrom.

14. The medical device (50) of claim 11, wherein the central opening (106b) is configured to receive elongate structure (104b) therethrough.

15. The medical device (50) of one of claims 13 or 14, wherein the arm (106c) is shaped for manipulation by an operator's thumb.

## Patentansprüche

1. Medizinische Vorrichtung (50), umfassend:
eine längliche Hülle (52) mit einem proximalen Ende (54), einem distalen Ende (56) und einem sich dazwischen erstreckenden Lumen (62), wobei mindestens ein Abschnitt eines Betätigungselements (112) innerhalb des Lumens (62) verschiebbar angeordnet ist, wobei ein distaler Abschnitt des Betätigungselements (112) funktionsbereit mit einem Endeffektor gekoppelt ist (68) und wobei ein Griff (100) am proximalen Ende (54) der länglichen Hülle (52) angeordnet ist und wobei der Griff (100) Folgendes umfasst:
einen länglichen Körperabschnitt, der eine zentrale Bohrung (108a) dort hindurch definiert, wobei die zentrale Bohrung (108a) eine proximale Öffnung beinhaltet, wobei ein distaler Abschnitt des länglichen Körperabschnitts funktionsbereit mit der länglichen Hülle (52) gekoppelt ist; und
einen Aktuator (104), der funktional mit dem Betätigungselement (112) gekoppelt ist und innerhalb der zentralen Bohrung (108b) des länglichen Körperabschnitts verschiebbar angeordnet ist, wobei der Aktuator (104) eine allgemein planare Oberfläche und eine längliche rohrförmige Struktur (104b), die davon abhängt und konfiguriert ist, um innerhalb der zentralen Bohrung (108a) aufgenommen zu werden, beinhaltet, wobei das Betätigungselement (112) konfiguriert ist, um innerhalb der länglichen rohrförmigen Struktur (104b) aufgenommen zu werden, wobei der Aktuator (104) konfiguriert ist, um zwischen einer ersten Längsposition relativ zu dem länglichen Körperabschnitt und einer von der ersten Längsposition verschiedenen zweiten Längsposition zu wechseln, wobei der Aktuator (104) fest an einen verstellbaren Körper (120) gekoppelt ist, der Gewinde auf einer äußeren Oberfläche davon beinhaltet, und wobei eine Oberfläche der zentralen Bohrung (108a) Gewinde zum Zusammenpassen mit den Gewinden auf dem verstellbaren Körper (120) beinhaltet.

2. Medizinische Vorrichtung (50) nach Anspruch 1, wobei die planare Oberfläche einen Durchmesser beinhaltet, der größer ist als ein Durchmesser der länglichen rohrförmigen Struktur (104b).

3. Medizinische Vorrichtung (50) nach Anspruch 1, wobei der Aktuator (104) konfiguriert ist, um sich bei Drehung des Aktuators (104) relativ zu dem länglichen Körperabschnitt in Längsrichtung relativ zu dem länglichen Körperabschnitt zu bewegen.

4. Medizinische Vorrichtung (50) nach Anspruch 1, wobei das Betätigungselement (112) an der allgemein planaren Oberfläche gesichert ist.

5. Medizinische Vorrichtung (50) nach Anspruch 1, wobei der längliche Körperabschnitt einen Sicherungsmechanismus zum Sichern des Griffs (100) an einem Abschnitt einer anderen medizinischen Vorrichtung beinhaltet und wobei der Sicherungsmechanismus eine Klammer (114) beinhaltet.

6. Medizinische Vorrichtung (50) nach Anspruch 1, wobei die andere medizinische Vorrichtung ein Endoskop beinhaltet.

7. Medizinische Vorrichtung (50) nach Anspruch 1, wobei ein proximaler Endabschnitt des Betätigungselements (112) fest an einem proximalen Ende des Aktuators (104) gesichert ist.

8. Medizinische Vorrichtung (50) nach Anspruch 5, wobei die Klammer (114) zwei nach außen hervorstehende Klammerarme (116) aufweist, um den Griff der medizinischen Vorrichtung sicher an der anderen medizinischen Vorrichtung zu befestigen.

9. Medizinische Vorrichtung (50) nach Anspruch 1, wobei der Aktuator (104) durch eine Feder (112) in die erste Längsposition verschoben ist.

10. Medizinische Vorrichtung (50) nach Anspruch 1, wobei die längliche Struktur (104b) des verstellbaren Körpers (120) eine Konfiguration, die der zentralen Bohrung(108a) entspricht, und ein Lumen, das sich darin erstreckt, um das Betätigungselement (112) aufzunehmen, aufweist.

11. Medizinische Vorrichtung (50) nach Anspruch 1, wobei der verstellbare Körper (120) an einem Kompensator (106) gesichert ist, der konfiguriert ist, um von einem Benutzer gedreht zu werden.

12. Medizinische Vorrichtung (50) nach Anspruch 11, wobei der Kompensator (106) und der verstellbare Körper (120) auf der länglichen Struktur (104b) des verstellbaren Körpers (120) aufgenommen sind.

13. Medizinische Vorrichtung (50) nach einem der Ansprüche 11 oder 12, wobei der Kompensator (106) einen unterlegscheibenartigen Abschnitt (106a) mit einer zentralen Öffnung (106b) und einem sich distal davon erstreckenden Arm (106c) beinhaltet.

14. Medizinische Vorrichtung (50) nach Anspruch 13, wobei die zentrale Öffnung (106b) konfiguriert ist, um die längliche Struktur (104b) dort hindurch aufzunehmen.

15. Medizinische Vorrichtung (50) nach einem der Ansprüche 13 oder 14, wobei der Arm (106c) zur Handhabung durch den Daumen eines Bedieners geformt ist.

## Revendications

1. Dispositif médical (50), comprenant :
une gaine allongée (52) qui comporte une extrémité proximale (54), une extrémité distale (56) et une lumière (62) qui s'étend entre ces deux extrémités, dans lequel au moins une partie d'un élément d'actionnement (112) est disposée de façon coulissante à l'intérieur de la lumière (62), dans lequel une partie distale de l'élément d'actionnement (112) est couplée de manière opérationnelle à un organe terminal effecteur (68) et dans lequel une poignée (100) est disposée au niveau de l'extrémité proximale (54) de la gaine allongée (52), et dans lequel la poignée (100) comprend :
une partie de corps allongée qui définit un alésage central (108a) au travers d'elle-même, dans lequel l'alésage central (108a) inclut une ouverture proximale, dans lequel une partie distale de la partie de corps allongée est couplée de manière opérationnelle à la gaine allongée (52) ; et
un actionneur (104) qui est couplé de manière opérationnelle à l'élément d'actionnement (112) et qui est disposé de façon coulissante à l'intérieur de l'alésage central (108b) de la partie de corps allongée, dans lequel l'actionneur (104) inclut une surface de forme générale plane et une structure tubulaire allongée (104b) qui en dépend et qui est configurée pour être reçue à l'intérieur de l'alésage central (108a), dans lequel l'élément d'actionnement (112) est configuré pour être reçu à l'intérieur de la structure tubulaire allongée (104b), dans lequel l'actionneur (104) est configuré pour réaliser une transition entre une première position longitudinale par rapport à la partie de corps allongée et une seconde position longitudinale qui est différente de la première position longitudinale ; dans lequel l'actionneur (104) est couplé de façon fixe à un corps réglable (120) qui inclut des filets sur sa surface externe et dans lequel une surface de l'alésage central (108a) inclut des filets destinés à s'accoupler avec les filets sur le corps réglable (120).

2. Dispositif médical (50) selon la revendication 1, dans lequel la surface de forme générale plane présente un diamètre qui est plus grand qu'un diamètre de la structure tubulaire allongée (104b).

3. Dispositif médical (50) selon la revendication 1, dans lequel l'actionneur (104) est configuré pour être déplacé de façon longitudinale par rapport à la partie de corps allongée suite à la rotation de l'actionneur (104) par rapport à la partie de corps allongée.

4. Dispositif médical (50) selon la revendication 1, dans lequel l'élément d'actionnement (112) est fixé de façon ferme et sécurisée sur la surface de forme générale plane.

5. Dispositif médical (50) selon la revendication 1, dans lequel la partie de corps allongée inclut un mécanisme de fixation ferme et sécurisée pour fixer de façon ferme et sécurisée la poignée (100) sur une partie d'un autre dispositif médical, et dans lequel le mécanisme de fixation ferme et sécurisée inclut un clip (114).

6. Dispositif médical (50) selon la revendication 1, dans lequel l'autre dispositif médical inclut un endoscope.

7. Dispositif médical (50) selon la revendication 1, dans lequel une partie d'extrémité proximale de l'élément d'actionnement (112) est fixée de façon ferme et sécurisée sur une extrémité proximale de l'actionneur (104).

8. Dispositif médical (50) selon la revendication 5, dans lequel le clip (114) comporte deux bras de clip (116) qui font saillie vers l'extérieur pour lier de façon ferme et sécurisée la poignée de dispositif médical à l'autre dispositif médical.

9. Dispositif médical (50) selon la revendication 1, dans lequel l'actionneur (104) est sollicité par poussée jusqu'à la première position longitudinale par un ressort (112).

10. Dispositif médical (50) selon la revendication 1, dans lequel la structure allongée (104b) du corps réglable (120) présente une configuration qui correspond à l'alésage central (108a) et comporte une lumière qui s'étend en son sein pour recevoir l'élément d'actionnement (112).

11. Dispositif médical (50) selon la revendication 1, dans lequel le corps réglable (120) est fixé de façon ferme et sécurisée à un compensateur (106) qui est configuré pour être entraîné en rotation par un utilisateur.

12. Dispositif médical (50) selon la revendication 11, dans lequel le compensateur (106) et le corps réglable (120) sont reçus sur la structure allongée (104b) du corps réglable (120).

13. Dispositif médical (50) selon l'une des revendications 11 ou 12, dans lequel le compensateur (106) inclut une partie similaire à une rondelle (106a) qui comporte une ouverture centrale (106b) et un bras (106c) qui s'étend de façon distale depuis.

14. Dispositif médical (50) selon la revendication 11, dans lequel l'ouverture centrale (106b) est configurée pour recevoir la structure allongée (104b) au travers.

15. Dispositif médical (50) selon l'une des revendications 13 ou 14, dans lequel le bras (106c) est conformé de sorte qu'il puisse être manipulé par le pouce d'un opérateur.
